# EUROPÄISCHE PATENTANMELDUNG

(11) **EP 0 725 064 A1**
(43) Veröffentlichungstag der Anmeldung: **07.08.1996**
(21) Anmeldenummer: 96100760.6
(22) Anmeldetag: 19.01.1996
(51) Int. Cl.: C07D 235/18, C07D 235/08, C07D 471/04, A61K 31/415, A61K 31/44

(54) **Verwendung von Phenylcyclohexylcarbonsäureamiden**

(30) Priorität: 01.02.1995 DE 19503160
(71) Anmelder: BAYER AG, 51368 Leverkusen (DE)
(72) Erfinder: Müller-Gliemann, Matthias, Dr., D-42697 Solingen (DE); Müller, Ulrich, Dr., D-42111 Wuppertal (DE); Beuck, Martin, Dr., Milford, CT 06460 (US); Zaiss, Siegfried, Dr., D-42113 Wuppertal (DE); Gerdes, Christoph, Dr., D-51373 Leverkusen (DE); Domdey-Bette, Anke, Dr., D-42499 Hückeswagen (DE); Grützmann, Rudi, Dr., D-42657 Solingen (DE); Lohmer, Stefan, Dr., I-20133 Milano (IT); Wohlfeil, Stefan, Dr., D-40724 Hilden (DE); Yalkinoglu, Özkan, Dr., D-42115 Wuppertal (DE); Elting, Özkan, Dr., Madison, CT 06443 (US); Denzer, Dirk, Dr., D-42115 Wuppertal (DE)

(57) **Zusammenfassung**

Die teilweise bekannten Phenylcyclohexylcarbonsäureamide eignen sich als Wirkstoffe in Arzneimitteln, insbesondere in Arzneimitteln zur Behandlung der Restenose.

## Beschreibung

Die Erfindung betrifft die neue Verwendung von Phenylcyclohexylcarbonsäureamiden, neue Stoffe, Verfahren zu ihrer Herstellung, insbesondere als Arzneimittel zur Behandlung von Restenose.

Es wurde gefunden, daß Phenylcyclohexylcarbonsäureamide der allgemeinen Formel (I), 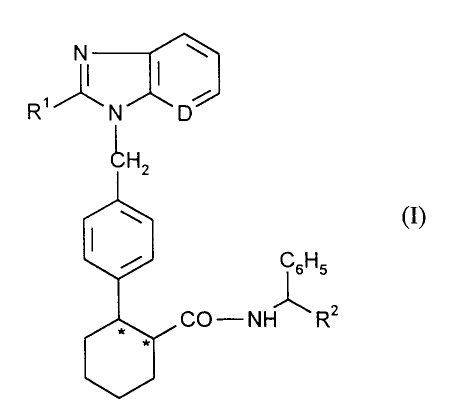
in welcher
- D: für die -CH-Gruppe oder ein Stickstoffatom steht,
- R¹: für Phenyl, Cycloalkyl mit 3 bis 6 Kohlenstoffatomen oder geradkettiges oder verzweigtes Alkyl mit bis zu 5 Kohlenstoffatomen steht,
- R²: für geradkettiges oder verzweigtes Alkoxycarbonyl mit bis zu 5 Kohlenstoffatomen oder für einen Rest der Formel -CO-NH₂ oder -CH₂-OH steht,
und deren Salze,
überraschenderweise eine starke Inhibition der Proliferation glatter Muskelzellen bewirken und somit geeignet sind zur Verwendung bei der Bekämpfung von Restenose.

Die Phenylcyclohexylcarbonsäureamide können erfindungsgemäß auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall-oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die Verbindungen können erfindungsgemäß in stereoisomeren Formen, die sich entweder wie Bild und Spiegelbild (Enantiomere), oder die sich nicht wie Bild und Spiegelbild (Diastereomere) verhalten, verwendet werden. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen.

Bevorzugt ist die Verwendung von Verbindungen der allgemeinen Formel (I),
in welcher
- D: für die -CH-Gruppe oder ein Stickstoffatom steht,
- R¹: für Phenyl, Cyclopropyl, Cyclopentyl, Cyclohexyl oder geradkettiges oder verzweigtes Alkyl mit bis zu 4 Kohlenstoffatomen steht,
- R²: für geradkettiges oder verzweigtes Alkoxycarbonyl mit bis zu 4 Kohlenstoffatomen steht oder für einen Rest der Formel -CO-NH₂ oder -CH₂-OH steht,
und deren Salze,
bei der Bekämpfung von Restenose.

Besonders bevorzugt ist die Verwendung von Verbindungen der allgemeinen Formel (I),
in welcher
- D: für die -CH-Gruppe oder ein Stickstoffatom steht,
- R¹: für Phenyl, Cyclopropyl, Ethyl oder iso-Propyl steht,
- R²: für geradkettiges oder verzweigtes Alkoxycarbonyl mit bis zu 4 Kohlenstoffatomen steht, oder für einen Rest der Formel -CO-NH₂ oder -CH₂-OH steht,
und deren Salze,
bei der Bekämpfung von Restenose.

Die Erfindung betrifft außerdem neue Verbindungen der allgemeinen Formel (I) mit den in der folgenden Tabelle angegebenen Substituentenbedeutungen:

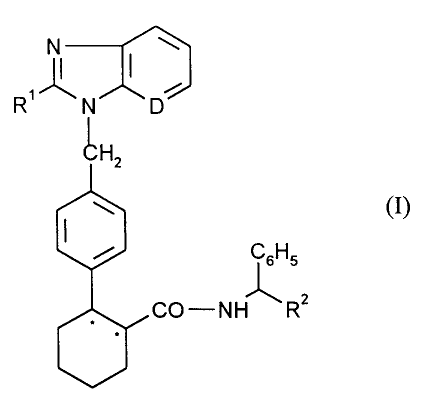

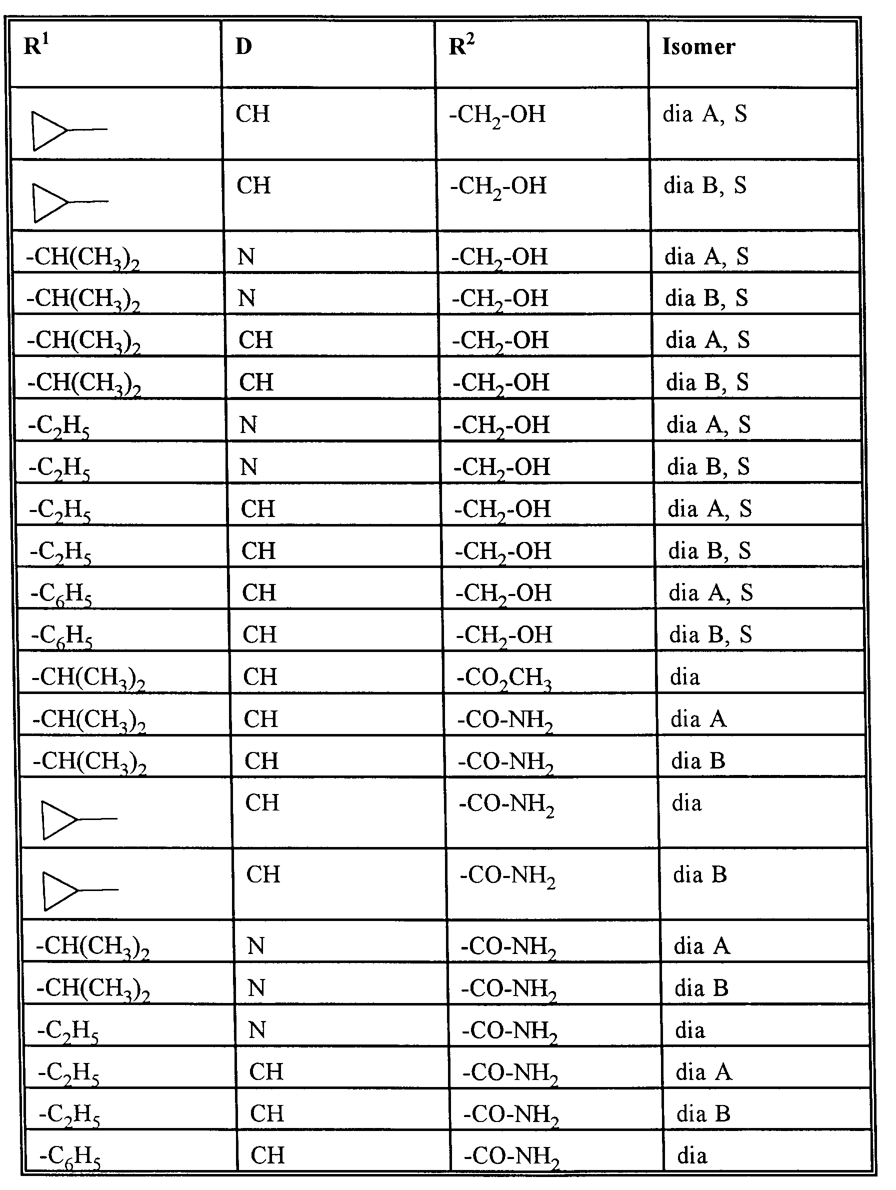

Die Verbindungen der allgemeinen Formel (I) werden hergestellt, indem man Verbindungen der allgemeinen Formel (II) 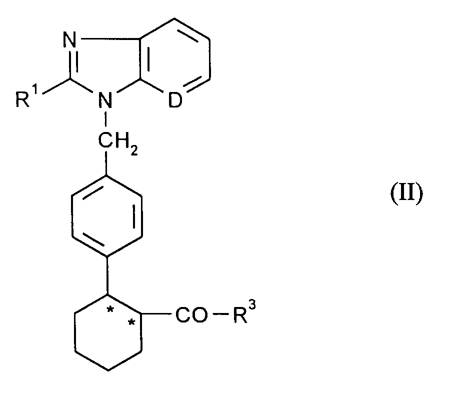
in welcher
- R¹ und D: die angegebene Bedeutung haben,
und
- R³: für geradkettiges oder verzweigtes C₁-C₄-Alkoxy steht,
verseift und die Säuren, gegebenenfalls unter vorgeschalteter Aktivierung, in inerten Lösemitteln, gegebenenfalls in Anwesenheit einer Base und/oder eines Dehydratisierungsmittel mit Phenylglycin-derivaten der allgemeinen Formeln (III) 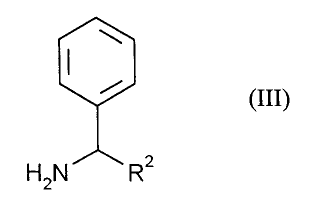
in welcher
- R²: die angegebene Bedeutung hat,
umsetzt,
und im Fall R²/R^{2'} = -CO-NH₂, gegebenenfalls ausgehend von den entsprechenden Estern eine Umsetzung mit Ammoniak in Alkoholen durchführt.

Das erfindungsgemäße Verfahren kann durch folgendes Formelschema beispielhaft erläutert werden: 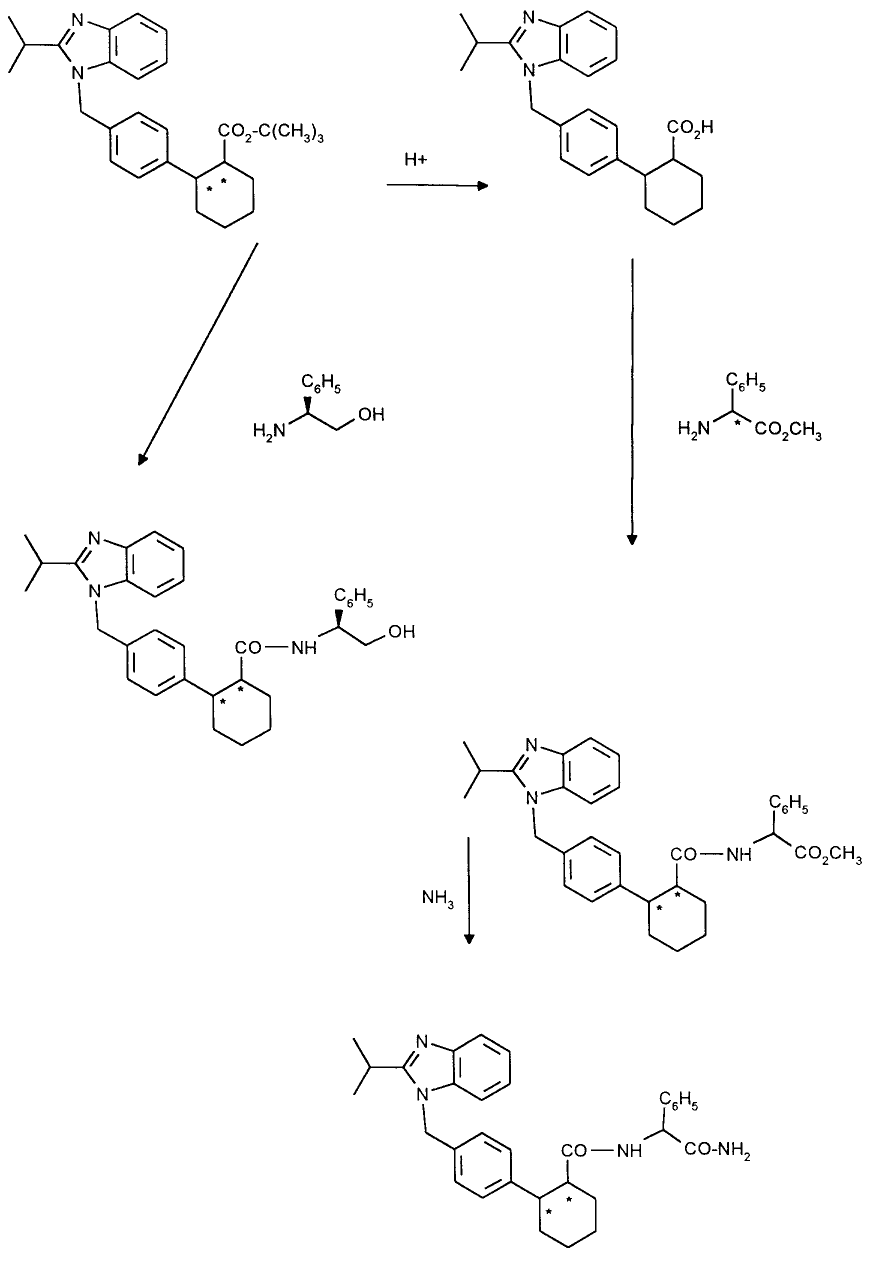
Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dichlormethan und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat.

Im allgemeinen setzt man die Base in einer Menge von 0,05 Mol bis 10 Mol, bevorzugt von 1 Mol bis 2 Mol bezogen auf 1 Mol der Verbindung der Formel (III) ein.

Das erfindungsgemäße Verfahren wird im allgemeinen in einem Temperaturbereich von -50°C bis +100°C, bevorzugt von -30°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Amidierung kann gegebenenfalls über die aktivierte Stufe der Säurehalogenide oder gemischter Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können, verlaufen.

Als Basen eignen sich dafür neben den oben aufgeführten Basen vorzugsweise Triethylamin und/oder Dimethylaminopyridin, DBU oder DABCO.

Die Base wird in einer Menge von 0,5 Mol bis 10 Mol, bevorzugt von 1 Mol bis 5 Mol, bezogen auf 1 Mol der Verbindungen der allgemeinen Formel (III) eingesetzt.

Als säurebindende Mittel für die Amidierung können Alkali-oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo-[4.3.0]-nonene-5 (DBN) oder 1,5-Diazabicyclo[5.4.0]undecene-7 (DBU) eingesetzt werden. Bevorzugt ist Triethylamin.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 Mol, bevorzugt von 1 bis 1,5 Mol, bezogen auf 1 Mol der entsprechenden Carbonsäuren, eingesetzt.

Die Überführung zu den entsprechenden Phenylglycinamidoamiden (R²/R^{2'} = -CONH₂) erfolgt mit alkoholischem Ammoniak, bevorzugt mit Ammoniak gesättigten Alkoholen, wie beispielsweise Methanol oder Ethanol, vorzugsweise Methanol, bei Raumtemperatur.

Die Verbindungen der allgemeinen Formel (III) sind bekannt oder können nach üblichen Methoden hergestellt werden.

Die Verbindungen der allgemeinen Formel (II) sind neu oder können durch Umsetzung von Verbindungen der Formel (IV) 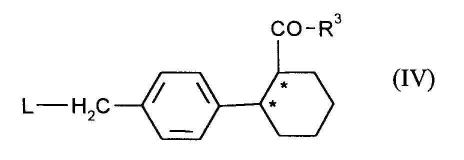
in welcher
- L: für eine typische Abgangsgruppe wie beispielsweise Chlor, Brom, Jod, Tosylat oder Mesylat, vorzugsweise für Brom, steht
und
- R³: die oben angegebene Bedeutung hat,
mit Verbindungen der allgemeinen Formel (V) 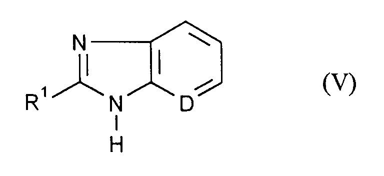
in welcher
- R¹ und D: die angegebene Bedeutungen haben,
in den oben angegebenen Lösemitteln, gegebenenfalls in Anwesenheit der oben genannten Basen hergestellt werden.

Die Verbindungen der allgemeinen Formel (I) zeigen überraschenderweise ein nicht vorhersehbares, wertvolles pharmakologisches Wirkspektrum.

Sie inhibieren die Proliferation von glatten Muskelzellen und können deshalb zur Behandlung der Restenose eingesetzt werden.

Die neuen Stoffe können darüber hinaus zur Behandlung der Arteriosklerose eingesetzt werden.

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Schweinen durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

**Tabelle A**

| Beispiel Nr. | IC₅₀ (nM) |
|---|---|
| 16 | 0,28 |
| 19 | 0,01 |

### Untersuchungen zur Inhibition der c-fos Genexpression glatter Muskelzellen durch die erfindungsgemäßen Verbindungen

Die antiproliferative Wirkung der Verbindungen wurde hinsichtlich der Serum- und Wachstumsfaktor-vermittelten Signaltransduktion und Induktion der c-fos Genexpression in Glattmuskelzell-Reporterlinien untersucht. Als Reporter dient hierbei die Luciferase, dessen Expression über den humanen c-fos Promotor gesteuert wird. Das c-fos Promotor/Luciferasekonstrukt ist stabil in die chromosomale DNA der Ratten-Glattmuskelzelllinie A 10 (ATCC CRL 1476) integriert. Die Reporterzellen werden in 96-Loch-Platten ausgesät und für 1-2 Tage in Serum-haltigem Medium (D-MEM mit 10% FCS, 2 mM L-Glutamin und 15 mM HEPES, pH 7,4) in 5% CO₂ bei 37°C kultiviert. Zur Unterdrückung der c-fos Promotoraktivität auf Basalwerte werden die Zellen durch Serumentzug für 24 Stunden arretiert. Anschließend werden Testverbindungen zugesetzt, und die Zellen mit FCS oder Wachstumsfaktoren zur Induktion der Luciferaseaktivität stimuliert. Nach dieser Behandlungsperiode (4 Stunden) werden die Zellen lysiert und deren Extrakte für die Luciferasebestimmung eingesetzt. Die IC₅₀-Werte werden aus der Wirkstoffkonzentration errechnet, die bei sequentieller Verdünnung des Wirkstoffes die halbmaximale Hemmung der durch den jeweiligen Stimulus hervorgerufene Luciferaseaktivität bewirkt.

### In-vivo-Untersuchungen zur Hemmung der vaskulären Glattmuskelzell-Proliferation im Modell der luftperfundierten Rattencarotis

Die in-vivo-Untersuchungen zur Hemmung der vaskulären Glattmuskelzell-Proliferation im Modell der luftperfundierten Rattencarotis erfolgten nach der leicht modifizierten Methode von Fishman et al. (Lab. Invest. 32, 339-351, 1975); die Operation der Tiere erfolgte unter Nembutal^{R}-Anästhesie. Die rechte Arteria carotis communis wird freigelegt und mit zwei Gefäßklemmen in einem caudal zu cranialen Abstand von ca. 1,5 cm abgeklemmt. Eine Kanüle wird am cranialen Ende dieses Gefäßsegmentes eingeführt, das caudale Ende wird durch den Stich mit einer Nadel perforiert. Nach Spülung mit physiologischer Kochsalzlösung wird ein Luftstrom (25 ml/min für 4 min) durch das Segment perfundiert. Anschließend werden die Klemmen entfernt, die Blutung wird unter leichtem Druck gestillt und das Operationsfeld mit Wundklammern verschlossen. Die Tiere werden acht Tage nach der Operation getötet, und es werden die zuvor luftperfundierten wie auch zur Kontrolle die korrespondierenden contralateralen Carotidensegmente entnommen.
Mit der Applikation der Testsubstanzen (p.o., i.v., i.p., s.c.) wurde zwei Tage vor der Operation begonnen, die Behandlung erfolgte dann über den gesamten Versuchszeitraum (Behandlungsdauer insgesamt: 10 Tage).
Die luftinduzierte Glattmuskelzellproliferation erfolgte mittels Bestimmung des DNA-Gehaltes der Carotidensegmente nach Helms et al. (DNA 43, 39-49, 1985). Dazu werden die Gefäßstücke mit Proteinase K enzymatisch verdaut, die DNA wird isoliert und mit Bisbenzimid fluorometrisch bestimmt (DNA aus Heringssperma als Standard). Der DNA-Gehalt der Gefäße wird schließlich in µg DNA pro mm Carotide angegeben.

Zur Feststellung der antiproliferativen Wirkung der erfindungsgemäßen Verbindungen wird Ratten ein Ballonkatheter in die Halsschlagader eingeführt, dieser aufgeblasen und die Innenseite des Blutgefäßes durch Bewegen des Katheters verletzt [Clowes A.W., et al., Lab. Invest. Vol. 49, No. 3, p. 327, 1983]. Diese Schädigung bewirkt eine neointimale Glattmuskelproliferation, die Stenosen verursacht. Das Ausmaß der Gefäßeinengung bei den Tieren wird nach ca. 2 Wochen durch histologische Aufarbeitung der Blutgefäße bestimmt, indem an Gefäßquerschnitten die Fläche des Proliferationsgewebes vermessen wird.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 20 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 50mg/kg, vorzugsweise 1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Lösungsmittel

| | |
|---|---|
| A = Dichlormethan : Methanol : Essigsäure | 9:1:0,1 |
| B = Dichlormethan : Methanol : Ammoniak | 9:1:0,1 |
| C = Dichlormethan : Methanol | 95:5 |

### Ausgangsverbindungen

### Beispiel I

### 2-Isopropyl-benzimidazol

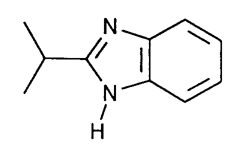

6,54 g (60 mMol) 2,3-Diaminopyridin, 5,6 g (60 mMol) Isobuttersäure und 45 ml Polyphosphorsäure werden zusammengegeben und 6 1/2 h auf 130°C erhitzt. Nach 6,5 h wird auf 0,4 l Eiswasser gegeben, mit fester NaOH auf pH 7 gebracht, Na₂CO₃ bis zum Ende der Gasentwicklung zugegeben und dreimal mit Essigester extrahiert. Die vereinigten organischen Phasen werden über Na₂SO₄ getrocknet und eingeengt.
- Ausbeute:: 8,45 g (87% d.Th.) R_{f} = 0,36 (CH₂Cl₂ / MeOH = 9/1)

### Beispiel II

### trans-2-[4-(2-Isopropylbenzimidazolyl-1H-methyl)phenyl]-cyclohexan-1-carbonsäure-tert.butylester

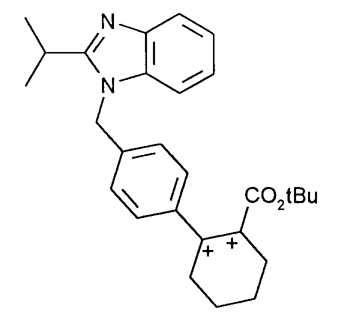

0,75 g NaH (25 mMol, 80%ig) wird in 50 ml DMF suspendiert, auf -10°C gekühlt und bei max. 0°C tropfenweise mit einer Lösung von 4,03 g (25 mMol) 2-Isopropylbenzimidazol in 60 ml DMF versetzt. Nach 15 min. Rühren wird bei max. 0°C eine Lösung von trans-2-(p-Brommethylphenyl)-cyclohexan-1-carbonsäure-tert.butylester in 110 ml DMF gegeben. Nach 2,5 h Rühren bei RT wird mit 25 ml 1 N HOAc versetzt und eingeengt. Der Rückstand wird in Ether/Wasser verteilt, die wäßrige Phase noch zweimal extrahiert, die vereinigte organische Phase über Na₂SO₄ getrocknet, eingeengt und das Produkt über Kieselgel 60 (PE/EE = 3/7) gereinigt.
- Ausbeute:: 2,91 g (28% d.Th.) R_{f} = 0,73 (CH₂Cl₂ / MeOH = 9/1)

### Beispiel III

### trans-2-[4-(2-Isopropylbenzimidazolyl-1H-methyl)-phenyl]-cyclohexancarbonsäure

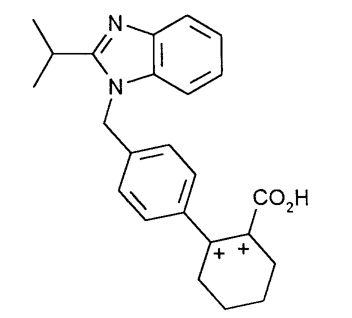

2,91 g (6,7 mMol) der Verbindung aus Beispiel II werden in 9 ml CH₂Cl₂ mit 9 ml Trifluoressigsäure bei RT 2,5 h gerührt. Nach Einengen wird zweimal in Ether aufgenommen und erneut eingeengt. Der Rückstand wird in Ether/H₂O (pH 10) verteilt, die wäßrige Phase sauer gestellt, noch dreimal extrahiert, und die vereinigten organischen Phasen werden zweimal mit H₂O/NH₄Cl, zweimal mit H₂O, einmal mit H₂O/NaHCO₃ und einmal mit H₂O/NH₄Cl geschüttelt, anschließend über Na₂SO₄ getrocknet und eingeengt.
- Ausbeute:: 2,2 g (86% d.Th.) R_{f} = 0,45 (CH₂Cl₂ / MeOH = 9/1)

### Herstellungsbeispiele

### Beispiel 1 und Beispiel 2

### trans-2-{4-[(2-Cyclopropyl-benzimidazo-1H-yl)-methyl]phenyl}-cyclohexan-1-carbonsäure-N-[(S)-phenylglycinol]amid

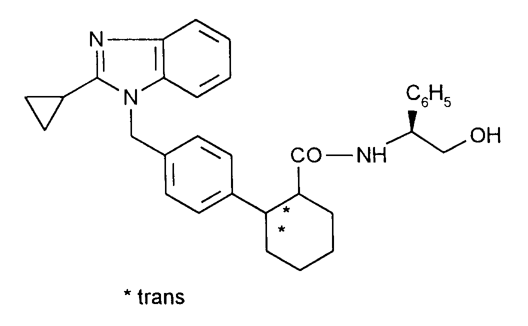

0,39 g (1 mMol) trans-2-{4-[(2-Cyclopropyl-benzimidazo-1H-yl)-methyl]phenyl}cyclohexan-1-carbonsäure werden zu einer Lösung von 0,14 g (1 mMol) L-Phenylglycinol in 10 ml CH₂Cl₂ gegeben, auf -10°C gekühlt und mit 0,23 g (1,2 mMol) N'-(3-Dimethylaminopropyl)-N-ethylcarbodiimid-hydrochlorid und 0,3 ml Triethylamin versetzt und über Nacht bei Raumtemperatur gerührt. Zur Aufarbeitung wird mit CH₂Cl₂ versetzt, je einmal mit gesättigter wäßriger Lösung von NH₄Cl, NaHCO₃ und (nach zusätzlichem Schütteln mit H₂O) NaCl geschüttelt. Nach Trocknen über Na₂SO₄ wird eingeengt und das Produkt chromatographiert (Kieselgel 60, CH₂Cl₂ / MeOH = 20/1).
- Ausbeute:: 0,16 g (0,32 mMol / R_{f} = 0,60 (B) dia A (Beispiel 1) 0,18 g (0,36 mMol / R_{f} = 0,50 (B) dia B (Beispiel 2)
In Analogie zur Vorschrift der Beispiele 1 und 2 werden die in der Tabelle 1 aufgeführten Verbindungen hergestellt:

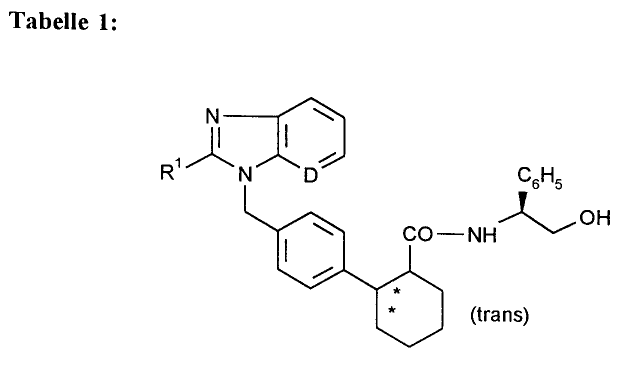

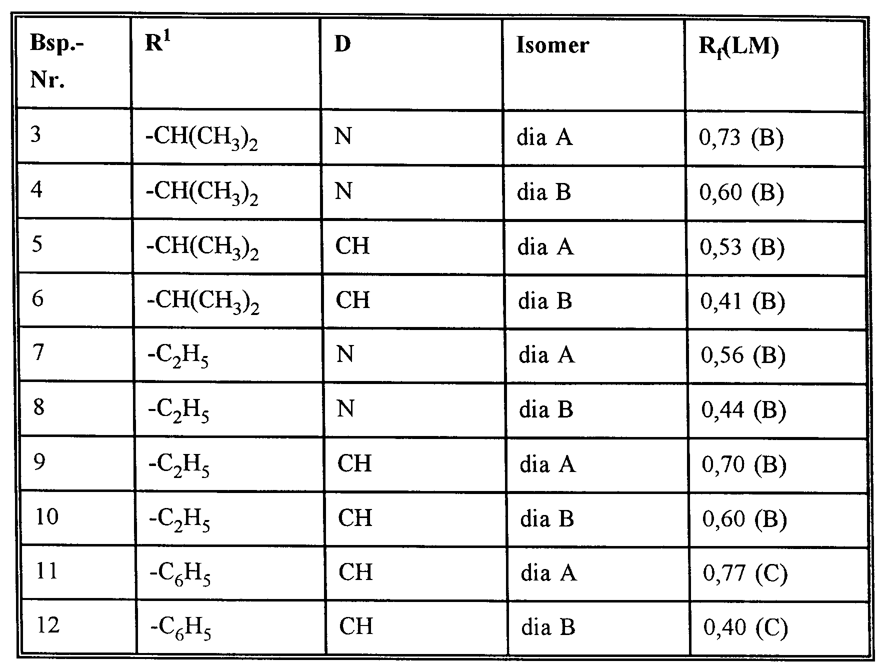

### Beispiel 13

### trans-2-[4-(2-Isopropylbenzimidazolyl-1H-methyl)phenyl]-cyclohexan-1-carbonyl-(phenylglycinmethylester)amid

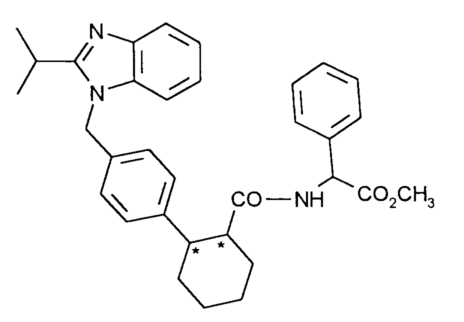

0,55 g (1,46 mmol) der Verbindung aus Beispiel III werden unter Argon bei -30°C gelöst, in 12 ml DMF mit 0,81 ml (5,82 mMol) Triethylamin und 0,12 ml (1,6 mMol) Mesylchlorid versetzt und 1 h gerührt. Anschließend wird eine Lösung von 0,35 g (1,7 mMol) Phenylglycinmethylester, 0,18 g (1,46 mMol) N,N-Dimethylaminopyridin und 0,24 ml (1,72 mMol) Triethylamin, gelöst in 10 ml DMF, zugetropft und über Nacht unter Rühren auf RT gebracht. Anschließend wird mit H₂O versetzt und dreimal mit Essigester extrahiert. Die vereinigten organischen Phasen werden über Na₂SO₄ getrocknet, eingeengt und das Produkt über Kieselgel 60 (CH₂Cl₂ / MeOH = 20/1) chromatographiert.
- Ausbeute:: 0,37 g (48% d.Th.) R_{f} = 0,51 (CH₂Cl₂ / MeOH = 95/5)

### Beispiel 14 und Beispiel 15

### trans-2-[4-(2-Isopropylbenzimidazolyl-1H-methyl)phenyl]cyclohexan-1-carbonyl-(phenylglycinamido)amid

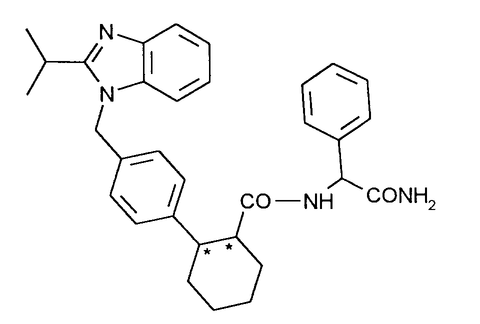

0,33 g (0,64 mMol) der Verbindung aus Beispiel 13 werden in 1 ml MeOH gelöst und mit 4 ml NH₃-gesättigtem MeOH 5 Tage bei RT gerührt. Anschließend wird der Niederschlag abgetrennt und die Lösung eingeengt. Der Rückstand wird über Kieselgel 60 (CH₂Cl₂ / MeOH / NH₃ = 100/5/0,3) gereinigt.
- Ausbeute:: 61 mg (19%d.Th.) dia A R_{f} = 0,36 (CH₂Cl₂/MeOH=9/1) Beispiel 14 35 mg (11%d.Th.) dia B R_{f} = 0,32 (CH₂Cl₂/MeOH=9/1) Beispiel 15
In Analogie zur Vorschrift der Beispiele 14 und 15 werden die in der Tabelle 2 aufgeführten Verbindungen hergestellt:

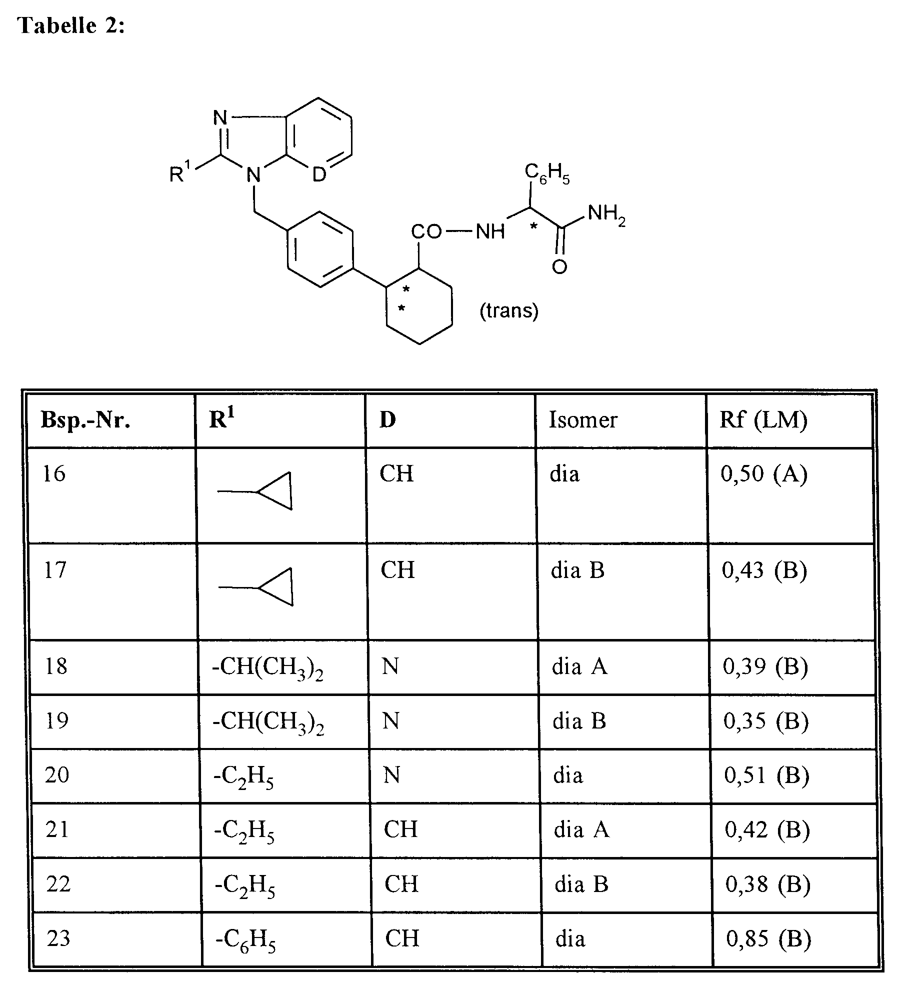

## Patentansprüche

1. Verwendung von Phenylcyclohexylcarbonsäureamiden der allgemeinen Formel (I) 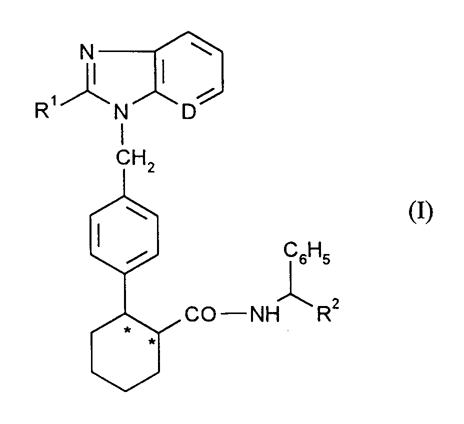 in welcher
D für die -CH-Gruppe oder ein Stickstoffatom steht,
R¹ für Phenyl, Cycloalkyl mit 3 bis 6 Kohlenstoffatomen oder geradkettiges oder verzweigtes Alkyl mit bis zu 5 Kohlenstoffatomen steht
R² für geradkettiges oder verzweigtes Alkoxycarbonyl mit bis zu 5 Kohlenstoffatomen oder für einen Rest der Formel -CO-NH₂ oder -CH₂-OH steht,
und deren Salze,
zur Herstellung von Arzneimitteln zur Behandlung von Restenose.

2. Verwendung von Phenylcyclohexylcarbonsäureamiden der Formel (I) nach Anspruch 1,
in welcher
D für die -CH-Gruppe oder ein Stickstoffatom steht,
R¹ für Phenyl, Cyclopropyl, Cyclopentyl, Cyclohexyl oder geradkettiges oder verzweigtes Alkyl mit bis zu 4 Kohlenstoffatomen steht,
R² für geradkettiges oder verzweigtes Alkoxycarbonyl mit bis zu 4 Kohlenstoffatomen steht oder für einen Rest der Formel -CO-NH₂ oder -CH₂-OH steht,
und deren Salze,
zur Herstellung von Arzneimitteln zur Behandlung der Restenose.

3. Verwendung von Phenylcyclohexylcarbonsäureamiden der Formel (I) nach Anspruch 1,
in welcher
D für die -CH-Gruppe oder ein Stickstoffatom steht,
R¹ für Phenyl, Cyclopropyl, Ethyl oder iso-Propyl steht,
R² für geradkettiges oder verzweigtes Alkoxycarbonyl mit bis zu 4 Kohlenstoffatomen steht, oder für einen Rest der Formel -CO-NH₂ oder -CH₂-OH steht,
und deren Salze,
zur Herstellung von Medikamenten zur Behandlung der Restenose.

4. Phenylcyclohexylcarbonsäureamide der Formel (I) nach Anspruch 1,
in denen die Substituenten R¹, R² und D die im folgenden angegebene Bedeutung haben: 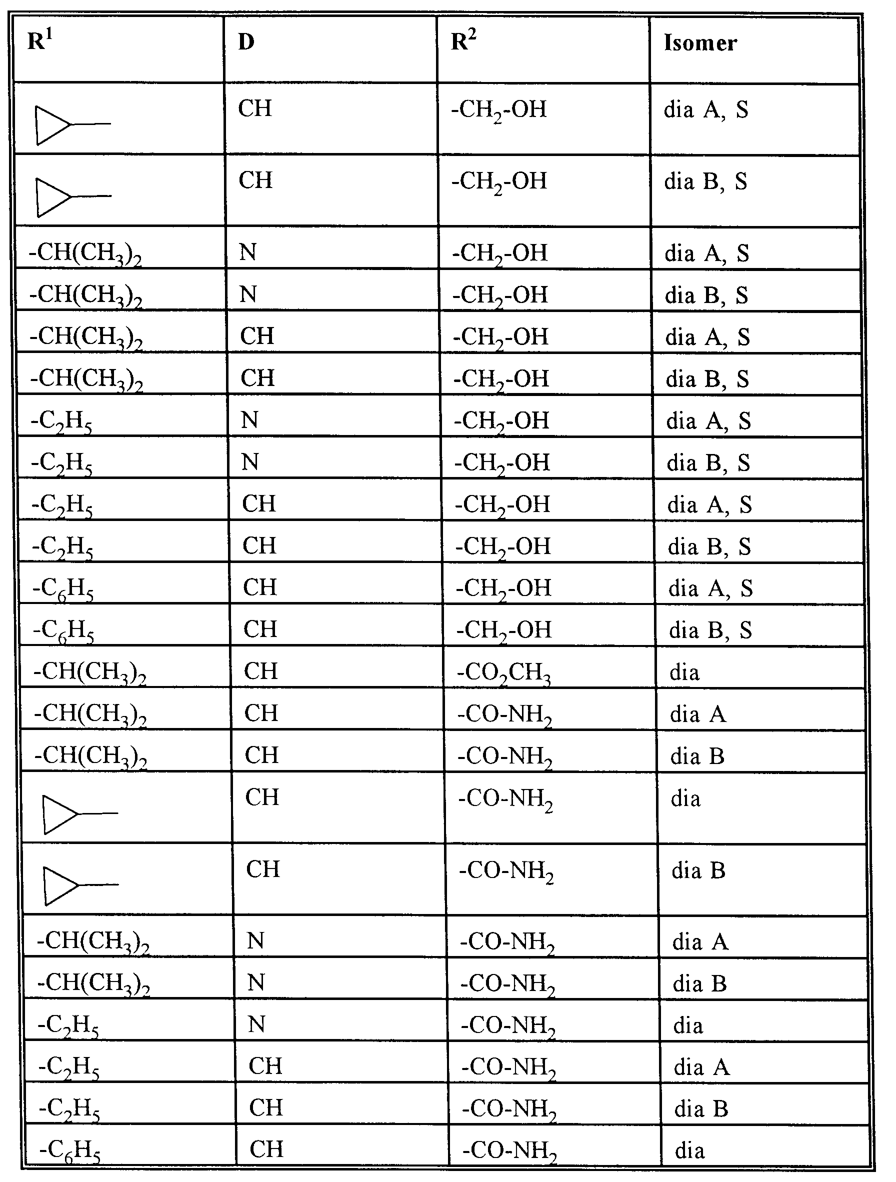 und deren Salze.

5. Verfahren zur Herstellung von Phenylcyclohexylcarbonsäureamiden nach Anspruch 4, dadurch gekennzeichnet, daß man Verbindungen der allgemeinen Formel (II) 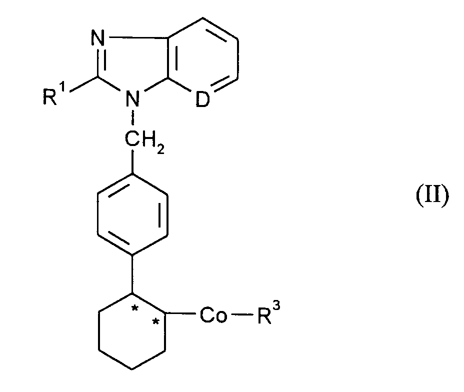 in welcher
R¹ und D die angegebene Bedeutung haben,
und
R³ für geradkettiges oder verzweigtes C₁-C₄-Alkoxy steht,
verseift und die Säuren, gegebenenfalls unter vorgeschalteter Aktivierung, in inerten Lösemitteln, gegebenenfalls in Anwesenheit einer Base und/oder eines Dehydratisierungsmittel mit Phenylglycin-derivaten der allgemeinen Formel (III) 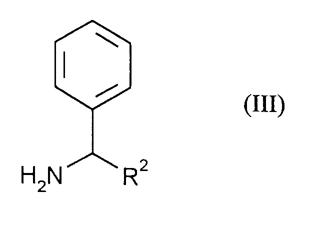 in welcher
R² die angegebene Bedeutung hat,
umsetzt,
und im Fall R²/R^{2'} = -CO-NH₂, gegebenenfalls ausgehend von den entsprechenden Estern eine Umsetzung mit ammoniakalischen Alkoholen durchführt.

6. Arzneimittel enthaltend Phenylcyclohexylcarbonsäureamide nach Anspruch 4.

7. Arzneimittel nach Anspruch 6 zur Behandlung der Restenose.

8. Verfahren zur Herstellung von Arzneimitteln nach Anspruch 6 bis 7, dadurch gekennzeichnet, daß die Phenylcyclohexylcarbonsäureamide, gegebenenfalls mit Hilfe von üblichen Hilfs- und Trägerstoffen in eine geeignete Applikationsform überführt werden.
